# EUROPÄISCHE PATENTSCHRIFT

(11) **EP 0 715 622 B1**
(45) Veröffentlichungstag und Bekanntmachung des Hinweises auf die Patenterteilung: **22.04.1998**
(21) Anmeldenummer: 94925456.9
(22) Anmeldetag: 13.08.1994
(51) Int. Cl.: C07D 231/12, C07B 35/04

(54) **VERFAHREN ZUR HERSTELLUNG VON PYRAZOL UND DESSEN DERIVATEN**
PROCESS FOR PREPARING PYRAZOLE AND ITS DERIVATIVES
PROCEDE DE PREPARATION DE PYRAZOLE ET DE SES DERIVES

(30) Priorität: 23.08.1993 DE 4328228
(43) Veröffentlichungstag der Anmeldung: 12.06.1996
(73) Patentinhaber: BASF AKTIENGESELLSCHAFT, 67056 Ludwigshafen (DE)
(72) Erfinder: MERKLE, Hans, Rupert, D-67061 Ludwigshafen (DE); FRETSCHNER, Erich, D-69239 Neckarsteinach (DE)
(86) Internationale Anmeldenummer: EP9402708
(87) Internationale Veröffentlichungsnummer: WO9506036

(56) Entgegenhaltungen:
- EP-A- 0 020 964
- EP-A- 0 366 328
- EP-A- 0 402 722
- EP-A- 0 474 037
- KATRITZKY A.R. & BOULTON A.J. 'ADVANCES IN HETEROCYCLIC CHEMISTRY' 1966 , VOL. 6 ; ACADEMIC PRESS , NEW YORK & LONDON Kost A.N. & Grandberg I.I. 'Progress in Pyrazole Chemistry' siehe Seite 358 - Seite 389
- WEISSBERGER A. 'THE CHEMISTRY OF HETEROCYCLIC COMPOUNDS' 1967 , VOL. 22 ; INTERSCIENCE PUBLISHERS , NEW YORK , LONDON , SYDNEY Wiley R.H. 'Pyrazoles, pyrazolines, pyrazolidines, indazoles and condensed rings' Part 1, Fusco R. 'Syntheses from pyrazolines by oxidation or other reactions' siehe Seite 41 - Seite 49

## Beschreibung

Die vorliegende Erfindung betrifft ein Verfahren zur Herstellung von Pyrazol und dessen Derivaten der Formel I 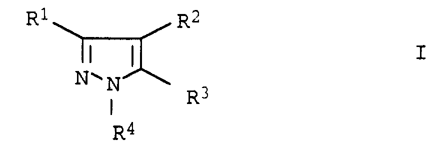 in der die Reste R¹, R², R³ und R⁴ für Wasserstoff oder C-organische Reste stehen, aus alpha,beta-ungesättigten Carbonylverbindungen der Formel II 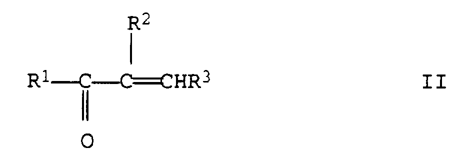 und Hydrazin oder Hydrazinderivaten der Formel III

H₂N-NHR⁴ III

Aus der EP-A 402 722 ist die Herstellung von Pyrazol und dessen Derivaten aus alpha,beta-ungesättigten Carbonylverbindungen und Hydrazin oder Hydrazinderivaten bekannt. In dem dort beschriebenen Verfahren wird entweder Pyrazolin oder eine Carbonylverbindung und ein Hydrazinderivat in einer Mischung von Schwefelsäure und Jod oder einer Jod oder Jodwasserstoff freisetzenden Verbindung in situ zum gewünschten Pyrazol umgesetzt. Die Ausbeuten liegen nach den beschriebenen Beispielen im Mittel um 78 % bezogen auf das eingesetzte Hydrazinderivat.

EP-A 020 964 beschreibt eine Synthese von Pyrazol(derivaten), wobei 1,3-Dicarbonylverbindungen mit Hydrazin(derivaten) in Gegenwart von Wasser und Säure umgesetzt werden.

EP-A 366 328 umfaßt die Umsetzung von 2,3-Dichlor-2-methylpropanal mit Hydrazin(derivaten) zu Pyrazol(derivaten).

In EP-A 474 037 ist die Synthese von 3(5)-Methylpyrazol(derivaten) ausgehend von Butendiol(derivaten) oder Ethinylalkylcarbinol(derivaten) und Hydrazin(derivaten) in Anwesenheit von Schwefelsäure und Jod beschrieben.

Katritzky und Boulton, Advances in Heterocyclic Chemistry; Progress in Pyrazole Chemistry, 6 (1966), 358-389 haben Verfahren zur Synthese diverser Pyrazole ausgehend von Hydrazinen, aliphatischen Diazoverbindungen bzw. Pyrazolinen und durch Umwandlung von Heterozyklen zusammengefaßt.

Fusco (In: The Chemistry of Heterocyclic Compounds; Synthesis from Pyrazolines by Oxidation or other Reactions, 22 (1967), 41 - 49) beschreibt ebenfalls verschiedene Zugänge zu Pyrazolderivaten.

Der vorliegenden Erfindung lag die Aufgabe zugrunde, Pyrazol und dessen Derivate in einfacher Weise mit besseren Ausbeuten und in einer höheren Reinheit herzustellen.

Demgemäß wurde ein Verfahren zur Herstellung von Pyrazol und dessen Derivaten der Formel I 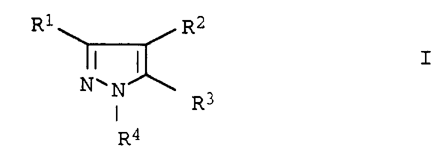 in der die Reste R¹, R², R³ und R⁴ für Wasserstoff oder C-organische Reste stehen, aus alpha,beta-ungesättigten Carbonylverbindungen der Formel II 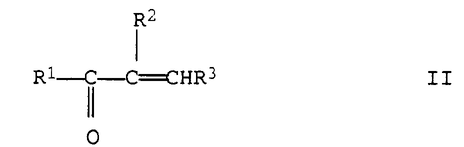 und Hydrazin oder Hydrazinderivaten der Formel III

H₂N-NHR⁴ III

gefunden, welches dadurch gekennzeichnet ist, daß man zunächst ohne zusätzliches Verdünnungsmittel und in Abwesenheit von Schwefelsäure als Reaktionsmedium eine alpha,beta-ungesättigte Carbonylverbindung der Formel II mit Hydrazin oder einem Hydrazinderivat der Formel III umsetzt und die dabei erhaltene Reaktionsmischung in einem weiteren Reaktionsschritt mit einer Mischung aus Schwefelsäure und Jod oder einer Jod oder Jodwasserstoff freisetzenden Verbindung umsetzt.

Bei diesem Verfahren wird zunächst eine alpha,beta-ungesättigte Carbonylverbindung II mit Hydrazin oder einem Hydrazinderivat III gemischt wobei während der Zumischung die Temperatur im Reaktionsmedium bei 0°C bis 100°C, vorzugsweise 10°C bis 70°C, insbesondere 20°C bis 50°C, gehalten wird. Da die Umsetzung von alpha,beta-ungesättigten Carbonylverbindungen II mit Hydrazin oder einem Hydrazinderivat III exotherm verläuft, kann es bei der Zumischung notwendig sein, die Reaktionsmischung zu kühlen. Bei der Vermischung ist es unerheblich, welchen der Reaktionspartner man vorlegt oder ob man die Ausgangsstoffe gleichzeitig aber getrennt in das Reaktionsvolumen einbringt. Zur Vervollständigung der Vermischung wird nach beendeter Zugabe üblicherweise noch 10 Minuten bis 60 Minuten bei der Mischungstemperatur nachgerührt.

Nach den bisherigen Erkenntnissen haben längere Nachrührzeiten keinen wesentlichen Einfluß auf die Vervollständigung der Umsetzung.

Die Ausgangsstoffe werden im allgemeinen in etwa stöchiometrischen Mengen miteinander umgesetzt, wobei das Verhältnis von Carbonylverbindung II zu Hydrazinderivat III üblicherweise 1:0,65 mol bis 1:1,25 mol beträgt. Ein anderes Verhältnis der Reaktionspartner beeinflußt den Ablauf der Umsetzung nicht wesentlich ist jedoch aus wirtschaftlichen Erwägungen nicht sinnvoll.

Hydrazin oder das Hydrazinderivat III können sowohl in Form der Hydrate oder der freien Basen als auch in Form von entsprechenden Hydrazoniumsalzen für das vorliegende Verfahren verwendet werden. Sofern Salze eingesetzt werden, die sich im Umsetzungsmedium nicht lösen, können durch ungenügende Vermischung Ausbeuteverluste auftreten. Bevorzugt verwendet man Hydrate oder die freien Basen der Hydrazinderivate III.

Das Verfahren beruht auf dem Prinzip, daß zunächst durch Umsetzung von Hydrazinen mit α,β-ungesättigten Carbonylverbindungen die entsprechenden Pyrazoline und Folgeprodukte gebildet werden. Die destillative Aufarbeitung nach Entfernung des Reaktionswassers liefert nur mäßige Ausbeuten, da neben den Pyrazolinen Nebenprodukte vorliegen, die durch Addition der Pyrazoline an die Ausgangscarbonylverbindungen entstehen und die ihrerseits mit den Hydrazinen Hydrazone und Azine bilden können.

Die so gewonnene Reaktionsmischung wird ohne weitere Aufarbeitung anschließend mit einer Mischung aus Schwefelsäure und Jod oder einer Jod oder Jodwasserstoff freisetzenden Verbindung umgesetzt. Hierbei geht man üblicherweise analog zu den Angaben aus der EP-A 402 722 vor, d.h. eine Mischung aus Schwefelsäure und Jod bzw. eine Jod oder Jodwasserstoff freisetzende Verbindung wird auf 50°C bis 250°C, vorzugsweise 70°C bis 200°C, insbesondere 100°C bis 180°C, erhitzt und bei dieser Temperatur mit der Reaktionsmischung aus der ersten Stufe versetzt.

Es ist auch möglich, die in der ersten Stufe erhaltene Reaktionsmischung bei niedrigeren Temperaturen, beispielsweise 10 bis 30°C in die Mischung aus Schwefelsäure und Jod oder einer Jod oder Jodwasserstoff freisetzenden Verbindung einzutragen. Aus verfahrenstechnischen Überlegungen heraus ist es jedoch vorteilhaft die Zugabe bei höherer Temperatur durchzuführen, da sich bei der Vermischung Salze bilden und die Reaktionsmischung schlechter gerührt werden kann. Bei welcher Temperatur die Zugabe erfolgt, hat nach bisherigen Erkenntnissen keinen Einfluß auf die Ausbeute der Umsetzung.

Diese zweite Stufe gehorcht wahrscheinlich im wesentlichen dem gleichen Prinzip wie die in der EP-A 402 722 beschriebene Umsetzung. Dementsprechend verwendet man im allgemeinen Schwefelsäure in einer Konzentration von nicht weniger als 30 Gew.-%. Üblicherweise wird 40 bis 99 Gew.-%ige Schwefelsäure, vorzugsweise 45 bis 95 Gew.-%ige Schwefelsäure, verwendet.

Die Menge an eingesetztem Jod oder der Jod oder Jodwasserstoff freisetzenden Verbindung bei dieser Umsetzung beträgt im allgemeinen 0.01 bis 10 mol-%, vorzugsweise 0.05 bis 5 mol-%, insbesondere 0.1 bis 2 mol-%, bezogen auf das Hydrazin bzw. das Hydrazinderivat III.

Neben Jod und Jodwasserstoff eignen sich als Jod oder Jodwasserstoff freisetzende Verbindungen beispielsweise auch Alkalimetall- und Erdalkalimetalljodide wie Lithiumjodid, Natriumjodid, Kalium-jodid, Cäsiumjodid, Magnesiumjodid und Calziumjodid sowie sonstige Metalljodide; es können grundsätzlich alle Jod- oder Jodwasserstoffverbindungen eingesetzt werden, die in der Lage sind unter den Reaktionsbedingungen Jod oder Jodwasserstoff freizusetzen. Hierzu zählen beispielsweise auch andere anorganische Jodverbindungen wie Alkalimetall-, Erdalkalimetall- oder andere Metallhypojodide, -jodite, -jodate und -perjodate oder organische Jodverbindungen, beispielsweise Alkyljodide wie Methyljodid.

Es hat sich gezeigt, daß für die Dehydrierungsreaktion bzw. die Oxidation des Jodids zu Jod die für die entsprechende Umsetzung optimale Temperatur von der Konzentration Schwefelsäure abhängt. Je geringer die Schwefelsäure konzentriert ist, umso höhere Umsetzungstemperaturen sind notwendig. Es empfiehlt sich daher während der Umsetzung das Reaktionswasser und das durch die Verwendung von Hydraten eingebrachte Wasser abzudestillieren um die Reaktionstemperatur niedrig zu halten.

Das der Reaktion entzogene Wasser enthält einen großen Teil des zugegebenen Jodids als Jod und Jodwasserstoff, welches nach Reduktion bzw. Neutralisation mit z.B. Natriumhydrogensulfit zurückgewonnen werden kann.

Nach beendeter Umsetzung läßt man das Reaktionsgemisch abkühlen, wobei das Pyrazolderivat im allgemeinen als Schwefelsäuresalz kristallisiert.

Zur Freisetzung des Pyrazols neutralisiert man das Reaktionsgemisch und extrahiert die neutrale Mischung mit einem inerten organischen, nicht mit Wasser mischbaren Lösungsmittel. Die organische Phase wird anschließend in üblicher Weise getrocknet und aufgearbeitet. Man erhält auf diese Weise Roh-Pyrazole in einer Reinheit von 85 bis 90 %, die durch einmalige Destillation auf einen Gehalt von 99 % gereinigt werden können.

Das vorstehende Verfahren eignet sich zur Herstellung von Pyrazol und dessen Derivaten der allgemeinen Formel I 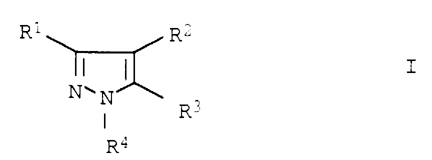 in der die Reste R¹, R², R³ und R⁴ unabhängig voneinander für Wasserstoff oder C-organische Reste stehen.

Als C-organische Reste sind zu nennen:
Alkylgruppen, welche geradkettig oder verzweigt sein können, beispielsweise C₁-C₁₀-Alkyl, vorzugsweise C₁-C₈-Alkyl, insbesondere C₁-C₆-Alkyl, wobei diese Reste ihrerseits durch Heteroatome wie Stickstoff, Sauerstoff und Schwefel unterbrochen sein können und Substituenten aus der folgenden Gruppe tragen können: Nitro, Carboxyl, Sulfonyl, Halogen, Cycloalkyl, Bicycloalkyl, Aryl und Hetaryl;
Cycloalkylgruppen oder Bicycloalkylgruppen, beispielsweise C₃-C₈-Cycloalkyl oder C₆-C₁₀-Bicycloalkyl, wobei diese Reste ihrerseits durch Heteroatome wie Stickstoff, Sauerstoff und Schwefel unterbrochen sein können und Substituenten aus der folgenden Gruppe tragen können: Nitro, Carboxyl, Sulfonyl, Halogen, Alkyl, Cycloalkyl, Bicycloalkyl, Aryl und Hetaryl;
Arylgruppen oder Hetarylgruppen wie Phenyl, Naphthyl und Pyridyl, wobei diese Reste ihrerseits Substituenten aus der folgenden Gruppe tragen können: Nitro, Carboxyl, Sulfonyl, Halogen, Alkyl, Cycloalkyl, Bicycloalkyl, Aryl und Hetaryl.

Insbesondere eignet sich das vorliegende Verfahren zur Herstellung von Pyrazolderivaten, bei denen mindestens einer der Reste R¹ bis R⁴ nicht Wasserstoff bedeutet.

Pyrazol und dessen Derivate finden beispielsweise Anwendung als Zwischenprodukte zur Herstellung von pharmakologisch wirksamen Verbindungen, Pflanzenschutzwirkstoffen oder auch Farbstoffen.

### Beispiele

1. Herstellung von 3-Methylpyrazol
   1.a Umsetzung von Crotonaldehyd und Hydrazinhydrat
      Zu 115 g (2,3 mol) Hydrazinhydrat wurden 169,1 g (2,415 mol) Crotonaldehyd gegeben, wobei die Temperatur durch Kühlung bei 30°C gehalten wurde. Nach Beendigung der Zugabe wurde die Mischung weitere 30 min. bei 25°C bis 30°C gerührt.
   1.b Umsetzung zu 3-Methylpyrazol
      Eine Mischung aus 720,8 g (5,06 mol) 68,8 %-iger Schwefelsäure und 0,76 g (5,1 mmol) Natriumjodid wurden auf 155°C erwärmt und bei dieser Temperatur mit der unter 1.a erhaltenen Mischung versetzt. Während der Zugabe und weitere 30 min. nach Ende der Zugabe wurde Wasser abdestilliert. Das hierbei abgetrennte Wasser wurde nach dem Abkühlen der Reaktionsmischung auf 70°C zur Verdünnung der Mischung wieder zugegeben.
      Die verdünnte Reaktionsmischung wurde mit 15%-iger Natronlauge auf einen pH-Wert von 8,5 bis 9 eingestellt. Bei dieser Neutralisation fiel ein großer Teil des Produktes als Öl an und kann durch decantieren abgetrennt werden. Durch Extraktion der wäßrigen Phase mit Isobutanol und anschließende destillative Aufarbeitung der gesammelten organischen Phasen erhielt man 172,5 g 3-Methylpyrazol (90,55 % bezogen auf das eingesetzte Hydrazinhydrat) von 99%-iger Reinheit (HPLC-Bestimmung). Kp.: 88°C/10 mbar.
2. Herstellung von 4-Methylpyrazol
   2.a Umsetzung von Methacrolein und Hydrazinhydrat
      Zu 115 g (2,3 mol) Hydrazinhydrat wurden 177,1 g (2,53 mol) Methacrolein gegeben, wobei die Temperatur durch Kühlung bei 30°C gehalten wurde. Nach Beendigung der Zugabe wurde die Mischung weitere 30 min. bei 25°C bis 30°C gerührt.
   2.b Umsetzung zu 4-Methylpyrazol
      Eine Mischung aus 720,8 g (5,06 mol) 68,8 %-iger Schwefelsäure und 1,00 g (6,7 mmol) Natriumjodid wurden auf 155°C erwärmt und bei dieser Temperatur mit der unter 1.a erhaltenen Mischung versetzt. Während der Zugabe und weitere 30 min nach Ende der Zugabe wurde Wasser abdestilliert. Das hierbei abgetrennte Wasser wurde nach dem Abkühlen der Reaktionsmischung auf 50°C zur Verdünnung der Mischung wieder zugegeben.
      Die verdünnte Reaktionsmischung wurde mit 15%-iger Natronlauge auf einen pH-Wert von 8,5 eingestellt. Bei dieser Neutralisation fiel ein großer Teil des Produktes als Öl an und kann durch decantieren abgetrennt werden. Durch Extraktion der wäßrigen Phase mit Isobutanol und anschließende destillative Aufarbeitung der gesammelten organischen Phasen erhielt man 170,5 g 4-Methylpyrazol (89,52 % bezogen auf das eingesetzte Hydrazinhydrat) von 99,2%-iger Reinheit (HPLC-Bestimmung). Kp.: 82°C/7 mbar.
3. Herstellung von 3,4-Dimethylpyrazol
   3.a Umsetzung von trans-2,3-Dimethylacrolein mit Hydrazinhydrat
      Zu 15,6 g (0,25 mol) Hydrazinhydrat 80 % wurden 22,1 g (0,2625 mol) trans 2,3-Dimethylacrolein gegeben, wobei die Temperatur durch Kühlung bei 30°C gehalten wurde. Nach Beendigung der Zugabe wurde die Mischung weitere 30 min. bei 25°C bis 30°C gerührt.
   3.b Umsetzung zu 3,4-Dimethylpyrazol
      Eine Mischung aus 74,2 g (0,52 mol) 68,8 %iger Schwefelsäure und 0,5 g (3,3 mmol) Natriumjodid wurde auf 155°C erwärmt und bei dieser Temperatur mit der unter 3.a erhaltenen Mischung versetzt. Während der Zugabe und weitere 30 min. nach Ende der Zugabe wurde Wasser abdestilliert. Das hierbei abgetrennte Wasser wurde nach dem Abkühlen der Reaktionsmischung auf 50°C zur Verdünnung wieder zugegeben.
      Die verdünnte Reaktionsmischung wurde mit 15 %iger Natronlauge auf einen pH-Wert von 8,5 eingestellt. Bei dieser Neutralisation fiel ein großer Teil des Produktes als Öl an, der durch dekantieren abgetrennt werden konnte. Durch Extraktion der wäßrigen Phase mit Isobutanol und anschließende destillative Aufarbeitung der gesammelten organischen Phasen erhielt man 21,4 g 3,4-Dimethylpyrazol (88,4 % bezogen auf das eingesetzte Hydrazinhydrat) von 99,2 %iger Reiheit. Kp.: 96°C/10 mbar.
4. Herstellung von 1,5-Dimethylpyrazol
   4.a Umsetzung von Crotonaldehyd mit Methylhydrazin
      Zu 92 g (2 mol) Methylhydrazin wurden 147 g (2,1 mol) Crotonaldehyd gegeben, wobei die Temperatur durch Kühlung bei 30°C gehalten wurde. Nach Beendigung der Zugabe wurde die Mischung weitere 30 min bei 25°C bis 30°C gerührt.
   4.b Umsetzung zu 1,5-Dimethylpyrazol
      Eine Mischung aus 626,7 g (4,4 mol) 68,8 %iger Schwefelsäure und 0,66 g (4,4 mmol) Natriumjodid wurde auf 155°C erwärmt und bei dieser Temperatur mit der unter 4.a erhaltenen Mischung versetzt. Während der Zugabe und weitere 30 min. nach Ende der Zugabe wurde Wasser abdestilliert. Das hierbei abgetrennte Wasser wurde nach dem Abkühlen der Reaktionsmischung auf 70°C zur Verdünnung der Mischung wieder zugegeben.
      Die verdünnte Reaktionsmischung wurde mit 15 %iger Natronlauge auf einen pH-Wert von 8,5 bis 9 eingestellt. Bei dieser Neutralisation fiel ein großer Teil des Produktes als Öl an, der durch dekantieren abgetrennt werden konnte. Durch Extraktion der wäßrigen Phase mit Isobutanol und anschließende destillative Aufarbeitung der gesammelten organischen Phasen erhielt man 167,8 g 1,5-Dimethylpyrazol (86,7 % bezogen auf das eingesetzte Methylhydrazin) von 99,2 %iger Reinheit. Kp.: 157°C/1013 mbar
5. Herstellung von 3-Methylpyrazol
   5.a Umsetzung von Crotonaldehyd mit Hydrazinhydrat
      Zu 125 g (2,0 mol) Hydrazinhydrat 80 % wurden 147 g (2,1 mol) Crotonaldehyd gegeben, wobei die Temperatur durch Kühlung bei 30°C gehalten wurde. Nach Beendigung der Zugabe wurde die Mischung weitere 30 min. bei 25°C bis 30°C gerührt.
   5.b Umsetzung zu 3-Methylpyrazol
      Eine Mischung aus 449,2 g (4,4 mol) 95 %iger Schwefelsäure und 0,66 g (4,4 mmol) Natriumjodid wurden bei 25°C mit der unter 5.a erhaltenen Mischung versetzt, wobei die Temperatur durch Kühlung bei 25°C gehalten wurde. Anschließend wurde das Reaktionsgemisch in 45 min. auf 125°C gebracht und 60 min. bei 125°C gehalten. Während des Aufheizens und während der Nachrührzeit wurde Wasser abdestilliert. Das hierbei abgetrennte Wasser wurde nach dem Abkühlen der Reaktionsmischung auf 70°C zur Verdünnung der Mischung wieder zugegeben.
      Die verdünnte Reaktionsmischung wurde mit 10 %iger Natronlauge auf einen pH-Wert von 8,5 bis 9 eingestellt. Bei dieser Neutralisation fiel ein großer Teil des Produktes als Öl an, der durch dekantieren abgetrennt werden konnte. Durch Extraktion der wäßrigen Phase mit Isobutanol und anschließende destillative Aufarbeitung der gesammelten organischen Phasen erhielt man 143,4 g 3-Methylpyrazol (87 % bezogen auf das eingesetzte Hydrazinhydrat) von 99,5 %iger Reinheit.
      Kp.: 88°C/1013 mbar
Vergleich des erfindungsgemäßen Verfahrens mit dem aus der EP-A- 402 722 bekannten Verfahren
A. Herstellung von 3-Methylpyrazol nach dem erfindungsgemäßen Verfahren
   A.1 Umsetzung von Crotonaldehyd und Hydrazinhydrat
      Zu 62,5 g (1,0 mol) Hydrazinhydrat 80 % wurden 73,5 g (1,05 mol) Crotonaldehyd gegeben, wobei die Temperatur durch Kühlung bei 30°C gehalten wurde. Nach Beendigung der Zugabe wurde die Mischung weitere 30 min. bei 25°C bis 30°C gerührt.
   A.2 Umsetzung zu 3-Methylpyrazol
      Eine Mischung aus 313,6 g (2,2 mol) 68,8 %-iger Schwefelsäure und 0,33 g (2,2 mmol) Natriumjodid wurden auf 155°C erwärmt und bei dieser Temperatur mit der unter 1.a erhaltenen Mischung versetzt. Während der Zugabe und weitere 30 min. nach Ende der Zugabe wurde Wasser abdestilliert. Das hierbei abgetrennte Wasser wurde nach dem Abkühlen der Reaktionsmischung wieder zugegeben.
      Die verdünnte Reaktionsmischung wurde mit 15%-iger Natronlauge auf einen pH-Wert von 8,5 eingestellt. Bei dieser Neutralisation fiel ein großer Teil des Produktes als Öl an und kann durch decantieren abgetrennt werden. Durch Extraktion der wäßrigen Phase mit Isobutanol und anschließende destillative Aufarbeitung der gesammelten organischen Phasen erhielt man 73,4 g 3-Methylpyrazol (89 % bezogen auf das eingesetzte Hydrazinhydrat) von 99,5%-iger Reinheit (HPLC-Bestimmung). Kp.: 88°C/10 mbar.
B Herstellung von 3-Methylpyrazol nach dem aus der EP-A 402 722 bekannten Verfahren
   B.1 Eine Mischung aus 313,6 g (2,2 mol) 68,8 %-iger Schwefelsäure und 0,33 g (2,2 mmol) Natriumjodid wurden auf 155°C erwärmt und bei dieser Temperatur gleichzeitig mit 62,5 g (1 mol) Hydrazinhydrat 80 % und 73,6 g (1,05 mol) Crotonaldehyd versetzt. Während der Zugabe und weitere 30 min. nach Ende der Zugabe wurde Wasser abdestilliert. Das hierbei abgetrennte Wasser wurde nach dem Abkühlen der Reaktionsmischung auf 50°C zur Verdünnung der Mischung wieder zugegeben.
   B.2 Die verdünnte Reaktionsmischung wurde mit 15%-iger Natronlauge auf einen pH-Wert von 8,5 eingestellt. Bei dieser Neutralisation fiel ein großer Teil des Produktes als Öl an, das durch decantieren abgetrennt werden konnte. Durch Extraktion der wäßrigen Phase mit Isobutanol und anschließende destillative Aufarbeitung der gesammelten organischen Phasen erhielt man 62,4 g 3-Methylpyrazol (75,7 % bezogen auf das eingesetzte Hydrazinhydrat) von 99,5%-iger Reinheit.
      Kp.: 88°C/10 mbar.

## Patentansprüche

1. Verfahren zur Herstellung von Pyrazol und dessen Derivaten der Formel I 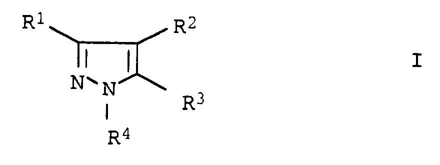 in der die Reste R¹, R², R³ und R⁴ für Wasserstoff oder C-organische Reste aus der Gruppe bestehend aus
Alkylgruppen, welche geradkettig oder verzweigt sein können, beispielsweise C₁-C₁₀-Alkyl, vorzugsweise C₁-C₈-Alkyl, insbesondere C₁-C₆-Alkyl, wobei diese Reste ihrerseits durch Heteroatome wie Stickstoff, Sauerstoff und Schwefel unterbrochen sein können und Substituenten aus der Gruppe Nitro, Carboxyl, Sulfonyl, Halogen, Cycloalkyl, Bicycloalkyl, Aryl und Hetaryl tragen können,
Cycloalkylgruppen oder Bicycloalkylgruppen, beispielsweise C₃-C₈-Cycloalkyl oder C₆-C₁₀-Bicycloalkyl, wobei diese Reste ihrerseits durch Heteroatome wie Stickstoff, Sauerstoff und Schwefel unterbrochen sein können und Substituenten aus der Gruppe Nitro, Carboxyl, Sulfonyl, Halogen, Alkyl, Cycloalkyl, Bicycloalkyl, Aryl und Hetaryl tragen können,
Arylgruppen oder Hetarylgruppen wie Phenyl, Naphthyl und Pyridyl, wobei diese Reste ihrerseits Substituenten aus der Gruppe Nitro, Carboxyl, Sulfonyl, Halogen, Alkyl, Cycloalkyl, Bicycloalkyl, Aryl und Hetaryl tragen können,
stehen,
aus alpha,beta-ungesättigten Carbonylverbindungen der Formel II 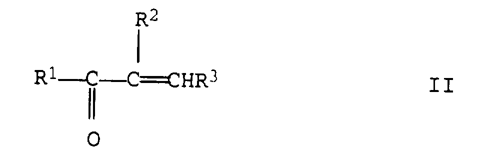 und Hydrazin oder Hydrazinderivaten der Formel III
H₂N-NHR⁴ III
dadurch gekennzeichnet, daß man zunächst ohne zusätzliches Verdünnungsmittel und in Abwesenheit von Schwefelsäure als Reaktionsmedium eine alpha,beta-ungesättigte Carbonylverbindung der Formel II mit Hydrazin oder einem Hydrazinderivat der Formel III umsetzt und die dabei erhaltene Reaktionsmischung in einem weiteren Reaktionsschritt mit einer Mischung aus Schwefelsäure und Jod oder einer Jod oder Jodwasserstoff freisetzenden Verbindung umsetzt.

2. Verfahren nach Anspruch 1, dadurch gekennzeichnet, daß man die Umsetzung der alpha,beta-ungesättigten Carbonylverbindung der Formel II mit Hydrazin oder einem Hydrazinderivat der Formel III bei Temperaturen von 0°C bis 100°C durchführt.

3. Verfahren nach Anspruch 1, dadurch gekennzeichnet, daß man 40 bis 99 Gew.-%ige Schwefelsäure verwendet.

4. Verfahren nach Anspruch 1, dadurch gekennzeichnet, daß man 0.01 bis 10 mol-% an Jod oder einer Jod oder Jodwasserstoff freisetzenden Verbindung bezogen auf das Hydrazin bzw. das Hydrazinderivat der Formel III verwendet.

5. Verfahren nach Anspruch 1, dadurch gekennzeichnet, daß man die aus der Umsetzung der alpha,beta-ungesättigten Carbonylverbindung der Formel II mit Hydrazin oder einem Hydrazinderivat der Formel III gewonnene Reaktionsmischung bei Temperaturen 50°C bis 250°C mit einer Mischung aus Schwefelsäure und Jod oder einer Jod oder Jodwasserstoff freisetzenden Verbindung umsetzt.

6. Verfahren nach Anspruch 1, dadurch gekennzeichnet, daß man während der Umsetzung der aus der Umsetzung der alpha,beta-ungesättigten Carbonylverbindung der Formel II mit Hydrazin oder einem Hydrazinderivat der Formel III gewonnenen Reaktionsmischung mit einer Mischung aus Schwefelsäure und Jod oder einer Jod oder Jodwasserstoff freisetzenden Verbindung das in der Mischung enthaltene Reaktionswasser entfernt.

## Claims

1. A process for preparing pyrazole and its derivatives of the formula I 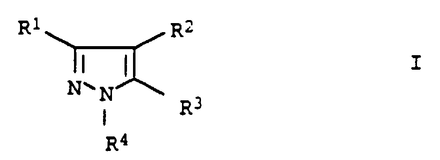 where R¹, R², R³ and R⁴ are each hydrogen or C-organic radicals from the group consisting of
alkyl groups which can be straight-chain or branched, for example C₁-C₁₀-alkyl, preferably C₁-C₈-alkyl, especially C₁-C₆-alkyl, it being possible for these radicals in turn to be interrupted by hetero atoms such as nitrogen, oxygen and sulfur and to carry substituents from the group nitro, carboxyl, sulfonyl, halogen, cycloalkyl, bicycloalkyl, aryl and hetaryl,
cycloalkyl groups or bicycloalkyl groups, for example C₃-C₈-cycloalkyl or C₆-C₁₀-bicycloalkyl, it being possible for these radicals in turn to be interrupted by hetero atoms such as nitrogen, oxygen and sulfur and to carry substituents from the group nitro, carboxyl, sulfonyl, halogen, alkyl, cycloalkyl, bicycloalkyl, aryl and hetaryl,
aryl groups or hetaryl groups such as phenyl, naphthyl and pyridyl, it being possible for these radicals in turn to carry substituents from the group nitro, carboxyl, sulfonyl, halogen, alkyl, cycloalkyl, bicycloalkyl, aryl and hetaryl,
from alpha,beta-unsaturated carboxyl compounds of the formula II 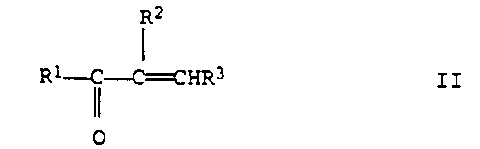 and hydrazine or hydrazine derivatives of the formula III
H₂N-NHR⁴ III
wherein, initially without additional diluent and in the absence of sulfuric acid as reaction medium, an alpha,beta-unsaturated carbonyl compound of the formula II is reacted with hydrazine or a hydrazine derivative of the formula III, and the resulting reaction mixture is reacted in another step with a mixture of sulfuric acid and iodine or a compound which liberates iodine or hydrogen iodide.

2. A process as claimed in claim 1, wherein the reaction of the alpha,beta-unsaturated carbonyl compound of the formula II with hydrazine or a hydrazine derivative of the formula III is carried out at from 0°C to 100°C.

3. A process as claimed in claim 1, wherein from 40 to 99% by weight of sulfuric acid is used.

4. A process as claimed in claim 1, wherein from 0.01 to 10 mol% of iodine or of a compound which liberates iodine or hydrogen iodide, based on the hydrazine or the hydrazine derivative of the formula III, is used.

5. A process as claimed in claim 1, wherein the reaction mixture obtained from the reaction of alpha,beta-unsaturated carbonyl compound of the formula II with hydrazine or a hydrazine derivative of the formula III is reacted at from 50°C to 250°C with a mixture of sulfuric acid and iodine or a compound which liberates iodine or hydrogen iodide.

6. A process as claimed in claim 1, wherein, during the reaction of the reaction mixture obtained from the reaction of the alpha,beta-unsaturated carbonyl compound of the formula II with hydrazine or a hydrazine derivative of the formula III with a mixture of sulfuric acid and iodine or a compound which liberates iodine or hydrogen iodide, the water of reaction present in the mixture is removed.

## Revendications

1. Procédé pour la préparation du pyrazole et de ses dérivés répondant à la formule I 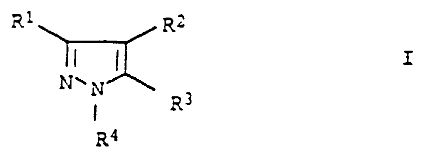 dans laquelle les symboles R¹, R², R³ et R⁴ représentent l'hydrogène ou des radicaux organiques carbonés choisis parmi
les groupes alkyle qui peuvent être à chaîne droite ou ramifiée, par exemple alkyle en C1-C10, de préférence alkyle en C1-C8 et plus spécialement alkyle en C1-C6, ces radicaux pouvant eux-mêmes être interrompus par des hétéroatomes tels que l'azote, l'oxygène et le soufre et pouvant porter des substituants choisis parmi les groupes nitro, carboxyle, sulfonyle, les halogènes, les groupes cycloalkyle, bicycloalkyle, aryle et hétéroaryle,
les groupes cycloalkyle ou bicycloalkyle, par exemple cycloalkyle en C3-C8 ou bicycloalkyle en C6-C10, ces radicaux pouvant eux-mêmes être interrompus par des hétéroatomes tels que l'azote, l'oxygène et le soufre et pouvant porter des substituants choisis parmi les groupes nitro, carboxyle, sulfonyle, les halogènes, les groupes alkyle, cycloalkyle, bicycloalkyle, aryle et hétéroaryle.
les groupes aryle ou hétéroaryle tels que phényle, naphtyle et pyridyle, ces groupes pouvant eux-mêmes porter des substituants choisis parmi les groupes nitro, carboxyle, sulfonyle, les halogènes, les groupes alkyle, cycloalkyle, bicycloalkyle, aryle et hétéroaryle,
à partir de dérivés carbonylés alpha, bêta-insaturés de formule II 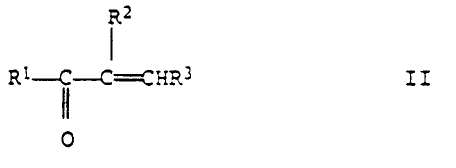 et de l'hydrazine ou de dérivés de l'hydrazine répondant à la formule III
H₂N-NHR⁴ III
caractérisé par le fait que l'on fait d'abord réagir, en l'absence d'un diluant étranger et sans utiliser l'acide sulfurique en tant que milieu de réaction, un dérivé carbonylé alpha,bêta-insaturé de formule II avec l'hydrazine ou un dérivé de l'hydrazine de formule III après quoi, dans un autre stade de réaction, on fait réagir le mélange obtenu au premier stade avec un mélange d'acide sulfurique et d'iode ou d'un composé libérant de l'iode ou de l'iodure d'hydrogène.

2. Procédé selon la revendication 1, caractérisé par le fait que l'on fait réagir le dérivé carbonylé alpha,bêta-insaturé de formule II avec l'hydrazine ou un dérivé de l'hydrazine de formule III à des températures de 0 à 100°C.

3. Procédé selon la revendication 1, caractérisé par le fait que l'on utilise de l'acide sulfurique à une concentration de 40 à 99 % en poids.

4. Procédé selon la revendication 1, caractérisé par le fait que l'on utilise de 0,01 à 10 mol % d'iode ou d'un composé libérant de l'iode ou de l'iodure d'hydrogène, par rapport à l'hydrazine ou au dérivé d'hydrazine de formule III.

5. Procédé selon la revendication 1, caractérisé par le fait que l'on fait réagir le mélange obtenu à la réaction entre le dérivé carbonylé alpha,bêta-insaturé de formule II et l'hydrazine ou un dérivé d'hydrazine de formule III avec un mélange d'acide sulfurique et d'iode ou d'un composé libérant de l'iode ou de l'iodure d'hydrogène à des températures de 50 à 250°C.

6. Procédé selon la revendication 1, caractérisé par le fait que, au cours de la réaction entre le mélange obtenu par réaction entre un dérivé carbonylé alpha,bêta-insaturé de formule II et l'hydrazine ou un dérivé de l'hydrazine de formule III et un mélange d'acide sulfurique et d'iode ou d'un composé libérant de l'iode ou de l'iodure d'hydrogène, on élimine l'eau de réaction contenue dans le mélange.
